Europäisches Patentamt

European Patent Office

Office européen des brevets

Numéro de publication: **0 284 697**

**A1**

# DEMANDE DE BREVET EUROPEEN

Numéro de dépôt: 87402798.0

Int. Cl.4: **C12M 1/12 , C12P 7/06**

Date de dépôt: **10.12.87**

Priorité: **12.12.86 FR 8617465**

Date de publication de la demande:
**05.10.88 Bulletin 88/40**

Etats contractants désignés:
**BE CH DE ES FR GB GR IT LI NL SE**

Demandeur: **Société pour l'Equipement des Industries Chimiques "SPEICHIM"**
**Tour Winterthur 102, Terrasse Boieldieu**
**F-92085 Paris-la Defense Cédex 18(FR)**

Demandeur: **Société Anonyme dite:**
**BEGHIN-SAY**

**F-59239 THUMERIES(FR)**

Inventeur: **GOMA, Gérard**
**20, Rue de Salas**
**F-31520 Ramonville-Saint Agne(FR)**
Inventeur: **PERLOT, Patrice**
**17, Avenue Emile Zola**
**F-31520 Ramonville-Saint Agne(FR)**
Inventeur: **MAZELLIER, Joel**
**4, Boulevard Griffoul Dorval**
**F-31400 Toulouse(FR)**

Mandataire: **Lepeudry-Gautherat, Thérèse et al**
**CABINET LEPEUDRY 6 rue du Faubourg St-Honoré**
**F-75008 Paris(FR)**

**Nouveau procédé utilisable en continu de fermentation par Saccharomyces cerevisiae d'un moût aqueux en vue de produire de l'éthanol et/ou unebiomasse de levure.**

L'invention concerne un nouveau procédé utilisable en continu de fermentation par Saccharomyces Cerevisiae dans un fermenteur (1) d'un moût aqueux (4) en vue d'obtenir de l'éthanol et/ou une biomasse de levure, selon lequel on fait circuler un flux de liqueur fermentée (6) à travers un organe séparateur (3) de façon à le diviser en un courant exempt de biomasse (9) et un courant recyclé (7) contenant toute la biomasse.

Selon l'invention l'organe séparateur (3) est un dispositif de filtration.

## Nouveau procédé utilisable en continu de fermentation par Saccharomyces cerevisiae d'un moût aqueux en vue de produire de l'éthanol et/ou une biomasse de levure.

La présente invention concerne un nouveau proédé de fermentation par Saccharomyces cerevisiae utilisable en continu dans un fermenteur d'un moût aqueux en vue d'obtenir de l'éthanol et/ou une biomasse de levure selon lequel on fait circuler un flux de liqueur fermentée à travers un organe séparateur de façon à le diviser en un courant exempt de biomasse, constitué d'un liquide appelé vin, et un courant recyclé contenant toute la biomasse.

Les procédés industriels classiques de fermentation en continu en vue de produire de l'éthanol, lesquels comportent un recyclage de la biomasse, sont mis en oeuvre dans des unités comportant un ensemble de fermenteurs disposés en série où la réaction de transformation des sucres est réalisée en deux étapes réactionnelles différentes et successives : production de biomasse favorisée dans les fermenteurs de tête et fermentation alcoolique proprement dite dans les fermenteurs suivants. A la sortie du dernier fermenteur les levures sont reprises, concentrées par centrifugation, lavées puis recyclées en tête de la cascade de fermentation.

Ces techniques sont décrites dans les demandes de brevet français publiées sous les n° 74 6078 et 77 0666.

Ces procédés classiques présentent des inconvénients, et notamment celui du temps de séjour important dans l'ensemble des fermenteurs nécessaire au bon déroulement de la fermentation.

Un procédé plus récent, qui est l'objet des demandes de brevet français n° 2 441 661 et 2 450 277 propose, dans le cadre plus général d'un - schéma complet de distillerie, un recyclage complémentaire vers le fermenteur des vinasses qui ont subi un épuisement en alcool après distillation du vin. Ce procédé "à deux recyclages" et un seul fermenteur permet de diminuer le temps de fermentation mais il présente l'inconvénient de produire un vin de teneur en éthanol de 6° au lieu de 8° GL dans les procédés classiques, ce qui nécessite un apport énergétique supplémentaire pour le distiller.

Il existe donc un besoin d'un procédé permettant d'opérer avec un temps de séjour plus court que les procédés classiques mais conservant leurs avantages, c'est-à-dire :
- une teneur en éthanol du vin au moins égale à 8° GL,
- une teneur en sucres résiduels fermentescibles nulle,
- un rendement de conversion des sucres en alcool égal ou supérieur à celui de procédés classiques,
- la possibilité de recycler tout ou partie des vinasses.

Les Demanderesses ont découvert avec surprise que l'utilisation d'un dispositif de filtration à la la place du dispositif de centrifugation des procédés classiques apportait de nombreux avantages et permettait un tel procédé.

L'invention concerne donc un nouveau procédé utilisable en continu de fermentation par Saccharomyces cerevisiae dans un fermenteur d'un moût aqueux en vue d'obtenir de l'éthanol et/ou une biomasse de levure, selon lequel on fait circuler un flux de liqueur fermentée à travers un organe séparateur de façon à le diviser en un courant exempt de biomasse et un courant recyclé contenant toute la biomasse, caractérisé en ce que l'organe séparateur est un dispositif de filtration.

On réalise le plus souvent ce procédé à l'aide d'un dispositif de microfiltration ou d'ultrafiltration tangentielles. Mais il est également possible d'utiliser tout autre dispositif de filtration qui permet de ne pas perdre de biomasse de façon continue dans le perméat (ou filtrat), tel que par exemple un dispositif où les premières particules déposées forment un gâteau qui empêche le passage ultérieur de cellules de levure.

La biomasse est retenue en permanence dans le fermenteur. En l'absence de purge la concentration en biomasse augmente mais la viabilité des cellules diminue. La concentration de biomasse active se stabilise à un niveau qui, en l'absence de purge, dépend uniquement des conditions du milieu de fermentation. L'utilisation d'une purge, qui soutire en permanence une certaine quantité du milieu de fermentation et donc de levure, permet un contrôle plus facile et plus souple de la concentration de biomasse. Cette purge assure de plus le renouvellement de la levure et contribue donc, par la maîtrise qu'elle apporte de son temps de séjour moyen dans le fermenteur, lequel, réglé par le débit de purge est avantageusement choisi inférieur à la durée de vie moyenne des cellules de levure, au maintien d'une viabilité (rapport du nombre de cellules vivantes sur le nombre total de cellules) élevée de la biomasse.

Dans le cas où on utilise ce procédé pour produire de l'éthanol il est intéressant d'envoyer le courant exempt de biomasse et la purge sur une colonne de distillation. Les vinasses obtenues au pied de la colonne sont alors, de préférence, en tout ou en partie recyclées vers le fermenteur.

On apprécie que le moût aqueux soit préparé à partir de mélasse de betterave ou de canne à sucre, d'égouts pauvres de 2ème jet de sucrerie (eau mère, souvent appelée "EP2", de la 2ème cristallisation en sucrerie ayant une teneur en matières sèches d'environ 68% et une pureté d'environ 77%) ou d'amidon saccharifié.

Le procédé de l'invention comporte de nombreux avantages, par rapport aux procédés classiques de production d'éthanol par fermentation et notamment celui de diminuer le temps nécessaire à la fermentation, ce qui permet de conduire celle-ci dans un fermenteur de volume raisonnable. Ce résultat est atteint grâce à l'emploi d'une concentration élevée de biomasse de levure dans des conditions qui permettent un excellent état physiologique de celle-ci et donc une croissance énergique et une activité satisfaisante de la levure vis-à-vis de la production d'éthanol, ce qui se traduit notamment par une productivité (masse d'éthanol produite par unité de temps et de volume) importante du système de fermentation.

Cette performance peut principalement s'expliquer par le fait inattendu, constaté expérimentalement comme exposé ci-après, que les cométabolites inhibiteurs de la fermentation de l'éthanol, substances toxiques métabolisées par les cellules en même temps que l'éthanol et bien connues de l'homme du métier, passent de façon préférentielle dans le perméat. Le courant porteur de la biomasse recyclé et donc le milieu de fermentation sont ainsi débarrassés de ces inhibiteurs toxiques, ce qui permet une bonne productivité en éthanol.

Cette détoxification favorise également une croissance énergique de la levure, ce qui permet d'atteindre une concentration élevée en biomasse dans le milieu de fermentation par ensemencement de ce dernier à l'aide de quantités relativement faibles de levure et éventuellement d'utiliser ce procédé en vue de produire de la levure.

Ce procédé présente en outre l'avantage, lequel contribue à l'excellent état physiologique de la biomasse, de ne pas provoquer, comme les procédés qui utilisent la centrifugation pour séparer la levure, une concentration brutale et excessive de biomasse, qui a pour effet de fatiguer la levure, notamment à cause de la déficience ainsi apportée en nutriments.

Un autre avantage important du procédé de l'invention réside dans sa mise en oeuvre sans l'aide de microorganismes manipulés génétiquement, lesquels sont coûteux et parfois peu stables. Il a été possible d'obtenir des résultats satisfaisants à l'aide de souches commerciales de Saccharomyces cerevisiae, notamment celles utilisées dans la levure de boulangerie.

L'invention sera mieux comprise à la lumière des résultats expérimentaux ci-après.

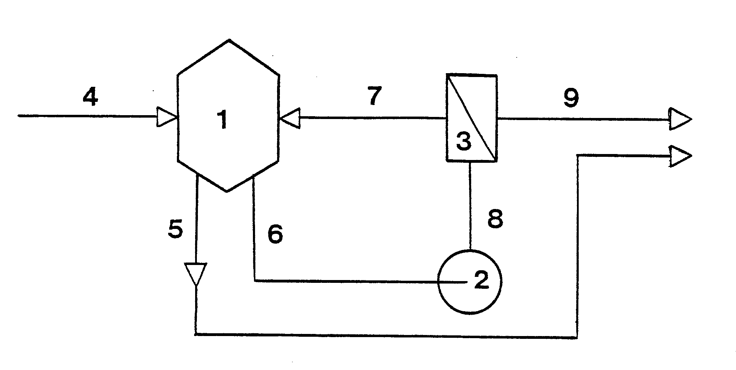

Ceux-ci ont été obtenus à l'aide d'un appareillage du type de celui qui est schématisé sur la figure unique et décrit ci-dessous.

Cet appareillage comprend un fermenteur constitué d'une cuve de fermentation 1 munie des accessoires habituels des fermenteurs, non représentés ici et notamment d'un agitateur mécanique ainsi que de dispositifs d'injection d'air, d'alimentation en solutions, (notamment salines), et d'extraction des gaz de fermentation, et une boucle de circulation comportant une pompe 2 et un dispositif de microfiltration tangentielle 3 reliés entre eux et au fermenteur par des tuyaux 6, 8 et 7.

Le moût, préparé selon un procédé connu, arrive dans la cuve de fermentation 1 par la tuyauterie 4. Le débit d'alimentation en moût est choisi en fonction du temps de séjour moyen souhaité dans le fermenteur. Les moûts fermentés sont soutirés par la tuyauterie 6 et repris par la pompe 2 qui les fait circuler dans la boucle de circulation à travers le dispositif de microfiltration tangentielle 3. Le vin est extrait par la tuyauterie 9 tandis que la partie contenant la levure débarrassée des cométabolites inhibiteurs mais à peine concentrée continue à circuler, retournant dans la cuve de fermentation 1 par la tuyauterie 7.

La tuyauterie de purge 5 permet le soutirage de la biomasse de la cuve de fermentation 1 à un débit choisi en fonction du temps de séjour moyen souhaité de la biomasse.

Le fermenteur est muni d'un dispositif d'aération : un débit d'air compris entre 3 et 4 volumes utiles du fermenteur par heure est généralement assuré dans le fermenteur. Lorsqu'on désire favoriser la croissance des cellules ce débit peut être porté à 20 volumes utiles du fermenteur par heure.

Les exemples 1 à 6 sont des exemples de réalisation de procédé de l'invention choisis pour aider l'homme du métier à comprendre l'invention, et en particulier, les possibilités et les avantages qu'elle offre ainsi que l'influence des différents paramètres. L'exemple 7 est un exemple de réalisation du procédé de l'invention qui donne à l'homme du métier des indications intéressantes sur la façon d'opérer pour mettre en oeuvre ce procédé dans le but de produire en continu de l'éthanol et en particulier stabiliser les différents paramètres à des valeurs intéressantes dans la pratique industrielle.

Dans tous les exemples, ci-après les concentrations de levure Saccharomyces cerevisiae données sont en levure comptée sèche et les concentrations de sucres sont exprimées en équivalent glucose, sauf spécifications contraires. Le temps de séjour est défini comme le rapport entre le volume utile dans la cuve de fermentation et la boucle de circulation -1 +6 +2 +8 +3 (partie rélentat) +7 et d'autre part le débit de moût entrant par la tuyauterie 4.

La température du milieu de fermentation est régulée entre 28 et 33°C.

EXEMPLE 1 : Mise en évidence du passage préférentiel des cométabolites inhibiteurs dans le perméat.

On ensemence à raison de quelques g/l de levure Saccharomyces cerevisiae un fermenteur de volume utile de 2,8 litres renfermant un moût à base de mélasse de betterave. On alimente ensuite en continu ce fermenteur par un moût constitué de mélasse de betterave diluée et clarifiée ayant une teneur en sucres totaux de 75 g/l.

Au bout d'un temps donné, on prélève simultanément un échantillon dans le fermenteur et un échantillon de vin clair à la sortie de l'organe de séparation. Les teneurs en alcool et en sucre résiduel des deux échantillons sont identiques. On extrait par centrifugation les levures contenues dans l'échantillon prélevé dans le fermenteur. On recharge les deux échantillons en sucre par addition de saccharose jusqu'à une concentration finale d'environ 80 g/l et on les complémente de solutions salines. On ensemence alors simultanément les deux échantillons avec la même quantité de levure de boulangerie.

Le milieu prélevé à la sortie de l'organe de séparation nécessite deux fois plus de temps pour fermenter que le milieu prélevé dans le fermenteur, ce qui prouve que les cométabolites inhibiteurs passent de façon préférentielle dans le perméat.

EXEMPLE 2 : Influence du débit d'alimentation en moût sur l'élimination des cométabolites toxiques inhibiteurs du milieu de fermentation.

On ensemence à raison de quelques g/l de levure Saccharomyces cerevisiae un fermenteur de volume utile de 2,3 litres renfermant un moût à base de mélasse de betterave. On alimente en continu ce fermenteur par un débit fixe d'un moût constitué de mélasse de betterave diluée et clarifiée ayant une concentration constante en sucres totaux comprise entre 20 et 70 g/l selon les essais.

On suit ensuite la concentration en biomasse dans le milieu de fermentation et on mesure le temps nécessaire pour atteindre une concentration en biomasse donnée.

On réitère l'ensemble des opérations décrites ci-dessus pour différentes valeurs des concentrations données en levure et des débits d'alimentation en moût. On obtient ainsi les résultats suivants.

Pour atteindre une concentration en biomasse de 15 g/l la durée nécessaire est de 47 h pour un débit correspondant à un temps de séjour de 11 h et de 4 h pour un temps de séjour de 30 mn. Pour atteindre une concentration en levure de 65 g/l les durées nécessaires sont de 30 h. 18 h et 9 h pour des temps de séjour respectifs de 1 h 40 mn. 30 mn et 15 mn.

Ces résultats montrent qu'un fort renouvellement du milieu de culture favorise la croissance des levures. Les cométabolites inhibiteurs toxiques sont d'autant mieux éliminés du milieu de fermentation, que le temps de séjour est faible, c'est-à-dire le débit d'alimentation en moût important.

EXEMPLE 3 : Possiblité de régler la concentration en biomasse à une valeur élevée.

On ensemence à raison de 5 g/l de levure Saccharomyces cerevisiae un fermenteur de volume utile de 2,8 litres renfermant un moût à base de mélasse de betterave. On alimente en continu ce fermenteur par un débit constant, correspondant à un temps de séjour de 55 mn, d'un moût constitué de mélasse de betterave diluée et clarifiée ayant une concentration de 50 g/l à 70 g/l de sucres totaux.

On suit l'évolution de la concentration en levure du moût dans le fermenteur. Cette concentration augmente progressivement pour atteindre au bout de 20 h une valeur de 150 g/l. La viabilité de la levure est alors proche de 100 %.

Le procédé permet donc d'atteindre des concentrations élevées en biomasse de haute viabilité qui peuvent être fixées à toute valeur jusqu'à 150 g/l.

EXEMPLE 4 : Possibilité de réduire le temps nécessaire à la fermentation grâce à l'emploi d'une concentration en biomasse élevée.

On ensemence à raison de quelques g/l de levure Saccharomyces cerevisiae un fermenteur de volume utile de 2,3 litres renfermant un moût à base de mélasse de betterave. On alimente en continu ce fermenteur par un débit correspondant à un temps de séjour de 3 h 15 mn de moût constitué de mélasse de betterave diluée et clarifiée ayant une concentration en sucres totaux de 125 g/l. La concentration en biomasse active et la teneur en éthanol du vin, augmentent progressivement et parallèlement jusqu'à des valeurs respectives de 25 g/l et 56 g/l, le vin produit étant alors épuisé en sucres fermentescibles.

Les performances suivantes ont été tenues pendant 7 jours : production en continu d'un vin à 56 g/l d'éthanol épuisé en sucres fermentescibles à partir de mélasse de betterave ayant une concentration en sucres totaux de 125 g/l, avec une concentration en biomasse active de 25 g/l, et un temps de séjour de 3 h 15 mn. La productivité correspondante est de 17 g d'éthanol par litre de milieu de fermentation et par heure.

En procédant de façon analogue les performances suivantes ont été tenues pendant 9 jours : production en continu d'un vin à 68 g/l d'éthanol épuisé en sucres fermentescibles à partir d'égouts pauvres de 2ème jet de sucrerie ayant une concentration en sucres totaux de 150 g/l, avec une concentration en biomasse de 40 g/l et un temps de séjour de 2 h 30 mn. La productivité correspondante s'élève à 27,2 g d'éthanol par litre de fermenteur et par heure.

La comparaison des performances ci-dessus avec celles des procédés de fermentation classiques - concentration de biomasse de l'ordre de 8 g/l et productivité d'environ 3,5 g/l par heure - montre l'intérêt de l'emploi d'une concentration élevée en biomasse. Celui-ci augmente la productivité et permet donc de réduire le temps de séjour du moût dans le fermenteur.

EXEMPLE 5 : Possibilité de contrôler la concentration en biomasse grâce à l'emploi d'une purge.

a) On ensemence à raison de 2 g/l de levure de boulangerie un fermenteur de volume utile de 2,8 litres renfermant un moût à base d'égout pauvre de 2ème jet de sucrerie. On alimente en continu le fermenteur, après une fermentation en discontinu, par un débit correspondant à un temps de séjour de 2 h de moût constitué d'égouts pauvres de 2ème jet dilué et clarifié ayant une concentration en sucres totaux de 147 g/l. La concentration en biomasse augmente progressivement pour atteindre la valeur de 72 g/l au bout de 24 heures de culture.

On décide alors de travailler avec une concentration en biomasse de 50 g/l. Dans ce but on extrait tout d'abord une partie du milieu de culture du fermenteur de façon à obtenir une concentration finale de biomasse de 50 g/l puis on met en place la purge à un débit choisi de façon à maintenir la concentration finale au niveau choisi en soutirant le surplus de biomasse formé.

Il a été ainsi possible de stabiliser la concentration de biomasse, à environ 50 g/l pendant 60 heures, ce qui a permis pendant cette période une productivité en éthanol d'environ 27,5 g par litre de fermenteur.

b) On ensemence à raison de 3 g/l de levure Saccharomyces cerevisiae un fermenteur d'un volume utile de 2,3 litres contenant un moût à base de mélasse de betterave. On alimente en continu ce fermenteur avec un moût constitué de mélasse de betterave diluée et clarifiée.

La mise en place d'une purge continue de levure sur le fermenteur, correspondant à 10 % du débit total de vin, dès le début du passage en continu de la fermentation conduit à la stabilisation de la concentration en levure à 30 g/l avec une viabilité supérieure ou égale à 80 % et à la production d'un vin à 7,5° avec un temps de séjour du moût de fermentation de 4 heures.

Ces exemples illustrent la possibilité de fixer, en choisissant correctement le débit de purge, la concentration de biomasse à la valeur désirée.

EXEMPLE 6 : Possibilité de recycler les vinasses sans changer les performances du procédé.

On ensemence à raison de quelques g/l de levure Saccharomyces cerevisiae un fermenteur de volume utile de 2,8 litres renfermant un moût à base de mélasse de betterave. On alimente ensuite en continu ce fermenteur par un débit de moût, correspondant à un temps de séjour de 4 h 10 mn, constitué de mélasse de betterave diluée et clarifiée d'une pureté (rapport en pourcentage des sucres totaux sur la somme de toutes les matières sèches solubles) de 59,9 %. Le vin séparé alimente une colonne à distiller de laboratoire. Les vinasses obtenues en pied de colonne sont épuisées en éthanol.

Après stabilisation de la teneur en éthanol du vin à 7° et fonctionnement pendant 7 jours, on recycle une partie des vinasses produites - environ 20 % de celles-ci - pour la dilution de la mélasse. La pureté du moût d'alimentation, plus chargé en matières sèches solubles que précédemment, est alors de 54,2 %.

Les performances du système, et en particulier, la teneur en alcool du vin produit ne sont pas affectées par le recyclage de 20 % des vinasses. Dans ces conditions, la production de vin épuisé en sucre fermentescible d'une teneur en éthanol de 7° avec un débit d'alimentation correspondant à un temps de séjour de 4 h 10 mn a été maintenue pendant 6 jours.

EXEMPLE 7 : Stabilisation des différents paramètres à des valeurs intéressantes au plan industriel dans le but de produire de l'éthanol.

On ensemence à raison de 5 g/l de levure Saccharomyces cerevisiae un fermenteur de volume utile de 10 m³ renfermant un moût préparé à partir d'égout pauvre de 2ème jet de sucrerie. On alimente ensuite en continu ce fermenteur par un débit d'alimentation de 4 m³ en moût constitué d'égout pauvre de 2ème jet de sucrerie ayant une concentration en sucres totaux de 50 g/l.

Le fermenteur est micro-aéré de façon à maintenir la levure dans un bon état physiologique.

On augmente ensuite progressivement au cours d'une période d'environ 30 h, la concentration en sucres fermentescibles jusqu'à 148 g/l. Au cours de cette phase on régule le pH entre 4 et 4,5. La concentration en levure du moût croît jusqu'à une valeur d'environ 45 g/l et le degré alcoolique du vin, constamment épuisé en sucre fermentescible, passe progressivement de 3° à 8,5°.

Quand ce paramètre atteint cette dernière valeur on met en place la purge à un débit d'environ 0,25 m³/h. On parvient ainsi, en maintenant le pH du milieu de fermentation à 4,5, à stabiliser l'ensemble des paramètres de la fermentation et notamment la concentration en biomasse de levure (à 45 g/l), la teneur en éthanol (à 8,5°) du vin produit, lequel est épuisé en sucre fermentescible, et le rendement de conversion en éthanol du sucre fermentescible. Ce dernier est légèrement supérieur à celui des procédés classiques.

**Revendications**

1. Procédé utilisable en continu de fermentation par Saccharomyces cerevisiae dans un fermenteur (1) d'un moût aqueux en vue d'obtenir de l'éthanol et/ou une biomasse de levure, selon lequel on fait circuler un flux de liqueur fermentée à travers un organe séparateur (3) de façon à le diviser en un courant exempt de biomasse et un courant recyclé contenant toute la biomasse, caractérisé en ce que l'organe séparateur (3) est un dispositif de filtration.

2. Procédé selon la revendication 1, caractérisé en ce que le dispositif de filtration est un dispositif de microfiltration tangentielle. .

3. Procédé selon la revendication 1, caractérisé en ce que le dispositif de filtration est un dispositif d'ultrafiltration tangentielle.

4. Procédé selon l'une quelconque des revendications précédentes, caractérisé en ce qu'on contrôle la concentration de biomasse dans le fermenteur à l'aide d'une purge.

5. Procédé selon la revendication 4, caractérisé en ce qu'on choisit le débit de purge de façon à ce que le temps de séjour moyen de la biomasse soit inférieur à la durée de vie moyenne des cellules de levure.

6. Procédé selon l'une quelconque des revendications précédentes, caractérisé en ce qu'on envoie le courant exempt de biomasse et la purge sur une colonne de distillation en vue de séparer l'éthanol.

7. Procédé selon la revendication 6, caractérisé en ce qu'on recycle vers le fermenteur en tout ou en partie les vinasses obtenues au pied de la colonne de distillation.

8. Application du procédé selon l'une quelconque des revendications précédentes à la production d'éthanol à partir d'un moût aqueux préparé à partir de mélasse de betterave et de canne à sucre d'égouts pauvres de deuxième jet de sucrerie ou d'amidon saccharifié.

9. Application du procédé selon l'une quelconque des revendications 1 à 5 à la production de levure.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 477 571 (UNISEARCH)<br>* Revendications 1,2,4; page 5, lignes 5-12,26-30; page 6, tableau 1; figure 1 *<br>--- | 1,2,8,9 | C 12 M 1/12<br>C 12 P 7/06 |
| X | EP-A-0 195 094 (STARCOSA)<br>* Revendications 1-4; page 4, lignes 14-24; exemple 1; figure 1 * | 1,2,4,8,9 | |
| Y | | 6,7 | |
| Y | WO-A-8 603 514 (J. GRANSTEDT)<br>* Revendication 1; figure 1 *<br>--- | 6,7 | |
| A | DE-A-3 005 605 (SARTORIUS)<br>* Revendications 1-3; figure 1 *<br>--- | 1-3 | |
| A | EP-A-0 065 895 (L'AIR LIQUIDE)<br>* Revendication 1; figure 1 *<br>----- | 1,9 | |

### DOMAINES TECHNIQUES RECHERCHES (Int. Cl.4)

C 12 M
C 12 P

Le présent rapport a été établi pour toutes les revendications

| Lieu de la recherche | Date d'achèvement de la recherche | Examinateur |
|---|---|---|
| LA HAYE | 21-03-1988 | WIESER, M.R.J. |